# EUROPEAN PATENT APPLICATION

(11) **EP 3 906 801 A1**
(43) Date of publication of application: **10.11.2021**
(21) Application number: 21172214.5
(22) Date of filing: 05.05.2021
(51) Int. Cl.: A43B 7/20

(54) **FOOTWEAR INVENTION**

(30) Priority: 05.05.2020 GB 202006628
(71) Applicant: Health Design Collective CIC, Musselburgh EH21 6UU (GB)
(72) Inventor: Robinson, Gillian, Margaret, Edinburgh, EH6 5RX (GB); Jagadamma, Kavi, Chandrasekharan, Musselburgh, EH21 6UU (GB); Bulley, Catherine, Joy, Musselburgh, EH21 6UU (GB); Santos, Derek, Francis, John, Musselburgh, EH21 6TS (GB)
(74) Representative: Cullen, Gavin James

(57) **Abstract**

A shoe comprising: a sole member comprising a toe portion, a ball portion and a heel portion; an ankle collar configured to be fixed to an ankle; a heel member configured to at least partially surround a heel of a foot, the heel member extending between the heel portion of the sole member and the ankle collar; one or more resilient members fixedly coupled between the ankle collar and substantially the ball portion of the sole member, wherein the one or more resilient members are biased against movement of the sole member away from the ankle collar and wherein the one or more resilient members are provided in a cross-over arrangement.

## Description

### Field

The present invention relates to an article of footwear, for example a shoe.

### Background

Foot drop is a condition that prevents or impedes dorsiflexion. Dorsiflexion is the ability to lift the front part of a foot and toes. There are a number of known ankle-foot orthoses (AFO) including splints and braces. Known splints are intended to hold the foot and ankle in a neutral position and are worn under socks and shoes.

Known problems with available splints and braces may include issues with comfort, as the splints and braces may cause swelling and/or become hot during use. A further known problem with these products is that their contact with the skin may cause skin problems. Furthermore, known splints and braces may be unattractive which may present a barrier to use.

Particular examples of known orthoses include the Aider Dropfoot Brace (Type 2 and Type 3), the Rebound foot-up, Swedish AFO and Step-on.

Known devices involves the process of placing a brace into a shoe which may be difficult, unnatural and cumbersome. Many known devices may also fail to achieve good movement and the end result may therefore be uncomfortable. Known devices may also necessitate the purchase of a pair of shoes bigger to accommodate the brace or splint.

### Summary

According to a first aspect of the present invention, there is provided an article of footwear comprising:
a sole member comprising a toe portion, a ball portion and a heel portion;
an ankle collar;
a heel member configured to at least partially surround a heel of a foot, the heel member extending between the heel portion of the sole member and the ankle collar;
one or more resilient members fixedly coupled between the ankle collar and substantially the ball portion of the sole member, wherein the one or more resilient members are biased against movement of the sole member away from the ankle collar.

The article of footwear may alleviate and/or reduce symptoms of a condition known as foot drop. The article of footwear may alleviate and/or reduce symptoms resulting from fatigue.

The movement of the sole member away from the ankle collar may comprise a relative movement of the sole member and the heel member thereby to increase an angle between the sole member and the heel member. The movement of the sole member may comprise rotation of the sole member relative to the heel member. The movement of the sole member may comprise one or more of flexing, bending and/or torsion of the sole member. The movement of the sole member may cause stretching and/or compression of the one or more resilient members. The one or more resilient members may prevent and/or resist movement of the sole member away from the ankle collar.

The movement of the sole member may comprise movement of the sole member from a rest configuration towards a further configuration such that in the rest configuration the one or more resilient members are at a first tension and such that, when held in the further configuration, the one or more resilient members are at a second, increased tension.

When the article of footwear is worn on a foot, the one or more resilient members of the article of footwear may be biased against movement of the sole member that results from the movement of the front part of the foot and/or the toes downwards such that the front part of the foot and/or toes is further from the ankle. When the article of footwear is worn on a foot, the one or more resilient members may be biased and/or resist movement of the sole member that results from the movement of the front part of the foot and/or the toes downwards such that the front part of the foot and/or toes would be further from the ankle without the resistance provided by the one or more resilient members.

The one or more resilient members may be biased against movements of the sole member that result from plantar flexion of the foot. The one or more resilient members may be biased against movements of the foot that are typical of a condition of the wearer, for example, foot drop. The one or more resilient members may be biased against movements of the foot that are typical of fatigue of the wearer.

The one or more resilient members may be provided in a cross-over arrangement. The cross-over arrangement may be such that the one or more resilient members cross-over at a cross-over position above the sole member. The cross-over position may be above an area between the ball portion and the heel portion. The cross-over position may be above the ball portion or above the heel portion.

In use, the cross-over arrangement may be such that the one or more resilient members cross-over at a cross-over position above a dorsal part of the foot, for example, a bridge of the foot. The one or more resilient members may conform to the shape of the foot such that the cross-over position, in use, is on an upper surface of the foot, for example, a bridge of the foot.

The one or more resilient members may comprise of or may comprise part of a mesh.

In use, the mesh may be provided at a dorsal part of the foot, for example, a bridge of the foot. In use, the mesh may be in contact with an upper surface of the foot.

The one or more resilient members may be provided such that, in response to a movement of the sole member away from the ankle collar, the one or more resilient members provide one or more forces opposing said movement.

The one or more resilient members may be arranged to disperse the one or more forces produced in response to movement of the sole member away from the ankle collar.

In comparison to providing a single resilient member in which any forces will act via a single point, the cross-over arrangement may provide an improved dispersion of forces. This may provide comfort benefits for the wearer. The cross-over arrangement may also prevent further foot movement during gait, for example, during inversion/eversion and/or adduction and abduction.

In use, the one or more produced forces may be in opposing directions at the cross-over position.

The one or more produced forces may be substantially along the one or more resilient members. The forces that prevent undesired movement of the foot may travel through the article of footwear rather than via the foot itself.

The one or more resilient members may be biased against lateral movement. The one or more resilient members may be biased against lateral movement of at least part of the sole member. The one or more resilient members may be biased against movement in a transverse plane. In use, the one or more resilient member may prevent or reduce adduction and abduction in a transverse plane.

The one or more resilient members may comprise at least two resilient members wherein a first resilient member exerts a first force in response to movement of the sole member away from the ankle collar and a second resilient member exerts a second force in response to movement of the sole member away from the ankle collar. The first force may be produced in a first direction and the second force may be produced in a second direction, wherein the first direction is substantially perpendicular to the second direction.

The first direction may form an angle with the second direction. The angle may be in the range of 0 to 180 degrees, for example, between 0 and 90 degrees or between 90 and 180 degrees. The angle may be in the range of 70 to 110 degrees or between 80 and 100 degrees or between 85 and 95 degrees. The angle may be an obtuse angle or an acute angle.

The first force may be exerted in a first direction from the front left side towards the upper rear right side of the article of footwear and the second force is exerted in a second direction from the front right side to the upper rear left side of the article of footwear. The first force may be exerted in a first direction from the front left side of the sole member towards the upper rear right side of the ankle collar and the second force is exerted in a second direction from the front right side of the sole member to the upper rear left side of the ankle collar of the article of footwear.

The one or more resilient members may be elastic. The one or more resilient members may comprise an elastic material.

The one or more resilient members may comprise medical grade elastic.

At least one of the ankle collar and/or at least one of the one or more resilient members may be adjustable. The adjustable ankle collar and/or at least one of the one or more resilient members may allow a wearer to compensate for over or under pronation during running.

The one or more resilient members may form part of a single continuous member that, in use, wraps at least in part around a wearers ankle. The single continuous member may comprise a first portion that is free to move, a second portion that is free to move and a third portion that is secured to the ankle collar.

The single continuous member may have at least a first portion characterized by a first stretch property and a second portion characterized by a second stretch property. The first portion may extends over the top of the foot, in use, and the second portion extends around the ankle, in use, and the first stretch property corresponds to a greater degree of stretch than the second stretch property.

The ankle collar may be configured to be fixed to an ankle such that the ankle collar is substantially static relative to the ankle during use. The ankle collar may provide an anchor point that is an integrated part of the article of footwear.

The heel member may have a length extending substantially perpendicular to the sole member such that the ankle collar is provided at a fixed distance from the heel member of the sole member wherein the length is such that, in use, the ankle collar is provided substantially at an ankle of a wearer.

The article of footwear may comprise one or more sole member couplings between the one or more resilient members and the sole member. The one or more sole member couplings may be provided at the ball region of the sole member.

The one or more sole-member couplings may be provided at the ball region of the sole member such that, in use, the sole-member coupling is at the ball of the foot.

The one or more sole-member couplings may comprise a first sole-member coupling between a first resilient member of the one or more resilient members to the sole member and a second sole-member coupling between a second resilient member of the one or more resilient members to the sole member, wherein the first sole-member coupling is provided at a first edge of the ball region of the sole member and the second sole-member coupling is provided at a second, opposing, edge of the ball region of the sole member.

The article of footwear may further comprise one or more ankle collar couplings between the one or more resilient members and the ankle collar, wherein the one or more ankle collar couplings are provided at one or more of a front, back and/or a side of the ankle collar.

The ankle collar may be a continuous structure. The ankle collar may form a complete loop. In use, the ankle collar may completely encircle the ankle.

The ankle collar may comprise a diameter and comprising adjustment mechanism for adjusting the diameter of the ankle collar. The ankle collar may have a length that is adjustable thereby to adjust the diameter of the ankle collar. The ankle collar may be configured to wrap around or coil around such that, in use, the diameter across the ankle collar is adjustable. The ankle collar diameter may be adjustable by wrapping or coiling the ankle collar around an ankle. The diameter of the ankle collar may be fixed by a securing mechanism.

The forces exerted by a resilient member may be between its sole-member couplings and its ankle collar couplings.

The one or more ankle collar couplings and/or sole-member couplings may comprise at least one of: a fixedly attached coupling; a detachable coupling; an adjustable coupling.

The article of footwear may be at least one of a shoe, a boot, a sandal, an athletic shoe, a running shoe and/or a hiking boot.

The sole member may comprise or form part of an inner sole of the article of footwear. The sole member may comprise or form part of an outer sole of the article of footwear. The one or more resilient members may comprise or may be integrated into an upper of the article of footwear. The one or more resilient members may comprise or form part of a saddle of the shoe. The ankle collar may be integrated into the upper of the article of footwear. The toe strap may comprise or form part of a toe cap. The ankle collar may comprise or provide part of a cuff or topline of the article of footwear. The heel portion may comprise or form part of the upper and/or a heel counter of the article of footwear. The heel portion may comprise or form part of an inner or an outer part of the article of footwear.

Each of the one or more resilient members may comprise an adjustable tension mechanism. The adjustable tension mechanism may allow the tension of each resilient member to be adjusted independently. The one or more resilient members may comprise two resilient members or two resilient member portions and each resilient member or resilient member portion may have a corresponding adjustable tension mechanism allowing the tension of the corresponding resilient member or resilient member portion to be adjusted independently. The adjustable tension mechanism may comprise a mechanism for adjusting the length of the resilient member, or resilient member portion, and for securing the resilient member at one or more points thereby to fix the un-stretched length of the resilient member. Such a mechanism may comprise, for example, buckle, clasp, zip, button, pin and toggle.

The ankle collar may substantially surround the ankle. The ankle collar may surround the ankle with a gap. The gap may be adjustable in size using an adjustment mechanism. The ankle collar may comprise or provides part of a cuff or topline of the shoe and the shoe may further comprises a further strap configured to be wrapped around an ankle, The further strap may provide reinforcement to the integrated the ankle collar. The integrated ankle collar may fix the shoe to the ankle. The further strap may fix the shoe to the ankle. The integrated ankle collar and further strap may together act as an anchor between the shoe and the ankle. The further strap and the one or more resilient members may form part of a single strap.

The ankle collar may be configured to be fixed to an ankle. The ankle collar may be fixedly attachable to the ankle. The ankle collar may act as an anchor between the ankle and the shoe. The size of the ankle collar may be adjustable, for example, to adjust the radius and/or length. An adjustment mechanism may be provided for adjusting the size of the ankle collar. The ankle collar may comprise a fixing mechanism for fixing the size of the ankle collar at a desired size. The desired size may be one in a continuum of sizes or one of a pre-set size. The choices of sizes may correspond to typical sizes of an ankle. The ankle collar may have an adjustment and/or fixing mechanism allowing a user to adjust the ankle collar to a desired size and then fix the ankle collar at the desired size.

According to a second aspect of the invention, there is provided an upper for an article of footwear, wherein the upper is configured to be attached to a sole member comprising a toe portion, a ball portion and a heel portion, wherein the upper comprises:
an ankle collar;
a heel member configured to partially surround a heel of a foot, wherein the heel member extends from the ankle collar and is configured to be attached to a heel portion of a sole member such that, when assembled, the heel member is extending between the heel portion of the sole member and the ankle collar; wherein the upper further comprises:
   one or more resilient members coupled to the ankle collar and configured to be coupled to substantially the ball portion of the sole member, such that, when assembled, the one or more resilient members are biased against movement of the sole member away from the ankle collar.

According to a third aspect of the invention, there is provided a kit of parts comprising, the upper in accordance with the second aspect of the invention and a sole member comprising a toe portion, a ball portion and a heel portion.

According to a fourth aspect of the invention, there is provided a method of assembling an article of footwear comprising:
providing an upper according to the second aspect,
providing a sole member comprising a toe portion, a ball portion and a heel portion;
attaching the upper to the sole member, wherein attaching the upper to the sole member comprises:
   attaching the heel member of the upper to the heel portion of the sole member;
   coupling the one or more resilient members to a ball portion of the sole member.

Any feature in one aspect of the invention may be applied to other aspects of the invention, in any appropriate combination.

### Brief Description of the Drawings

Various aspects of the invention will now be described by way of example only, and with reference to the accompanying drawings, of which:
Figure 1 is an illustration of an article of footwear, in accordance with a first embodiment;
Figure 2 is an illustration of an article of footwear, in accordance with a second embodiment;
Figures 3(a) and 3(b) are views of an article of footwear, in accordance with a third embodiment;
Figures 4(a) and 4(b) are views of an article of footwear, in accordance with a fourth embodiment;
Figures 5(a) to 5(c) are views of an article of footwear, in accordance with a fifth embodiment, and
Figure 6(a) and 6(b) are views of an article of footwear, in accordance with a sixth embodiment.

### Detailed Description of the Drawings

In accordance with embodiments, there is provided an article of footwear. The article of footwear may aid a sufferer or alleviate and/or reduce symptoms of a condition known as foot drop.

By way of background, it is known that a person moves in accordance with a (bipedal) gait cycle. A gait cycle is made up of strides and a typical stride will have a stance and a swing phase. The stance phase is the part of the cycle during which a reference foot remains in contact with the ground. The stance phase may be considered to comprise the following parts: Initial contact (for example, heel strike); Loading Response (foot flat); Mid Stance; Terminal Stance and Toe Off (Pre-swing). The swing phase is the part of the cycle in which the reference foot is not in contact with the ground. The swing phase has an initial swing, mid-swing and terminal swing.

Foot drop affects the ability of a patient to raise their foot at the ankle. Foot drop may be caused by muscular damage, nerve damage or signalling problems. Foot drop may be a symptom of a number of conditions including multiple sclerosis, stroke, spinal cord injury, diabetes and is caused by weakness in the ankle or disruption in the nerve pathway between the legs and the brain. People suffering from foot drop may adapt different gaits to compensate for foot drop. This may lead to further problems.

Foot drop is characterized by an inability to point the toes toward the body (dorsiflexion) due to weakness or paralysis of the muscles (dorsiflexors) that pull the foot up. In addition, there might be an inability to move the foot at the ankle inward (inversion) or outward (eversion). A foot drop gait cycle may have more exaggerated phases. For example, foot drop may affect the entire swing phase, as a patient with foot drop may have greater flexion at the knee to accommodate the inability to dorsiflex. In addition, with reference to the stance phase, at the initial contact of the reference foot, the foot may slap the ground or the entire foot may be planted on the ground at once, as opposed to a normal heel-toe foot strike. In addition, at the terminal stance and toe-off part, at which point the toe leaves the ground, a patient may not have the ability to support their body weight. The part of the gait cycle during which dorsiflexors are most active is from heel contact up to 20% of gait cycle, and again from just before toe-off continuing throughout the entire swing phase. Foot drop may also result in triplane motion due to the subtalar joint leading to adduction and abduction in the transverse plane.

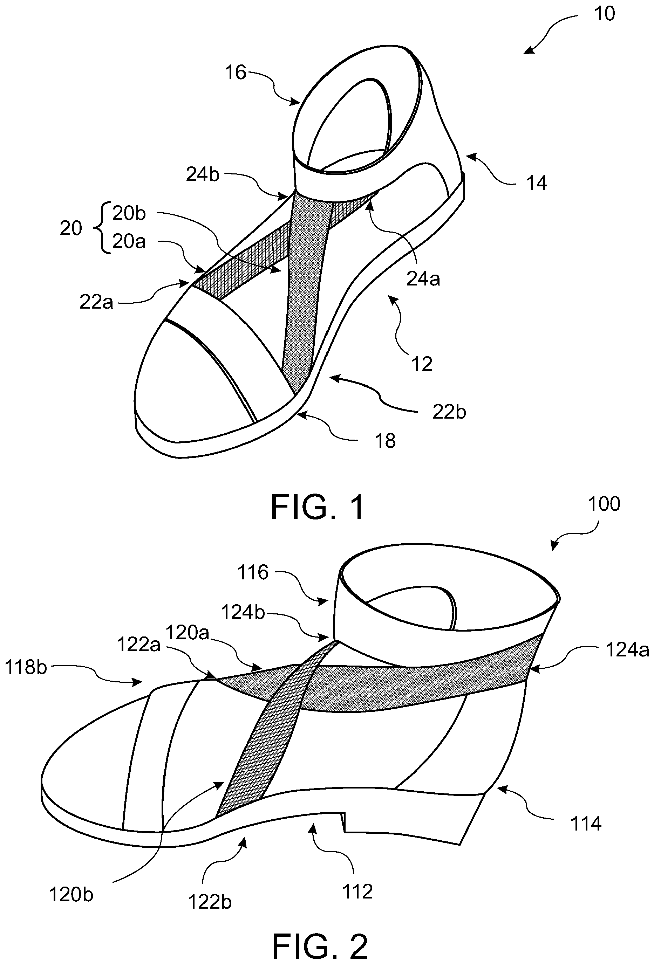

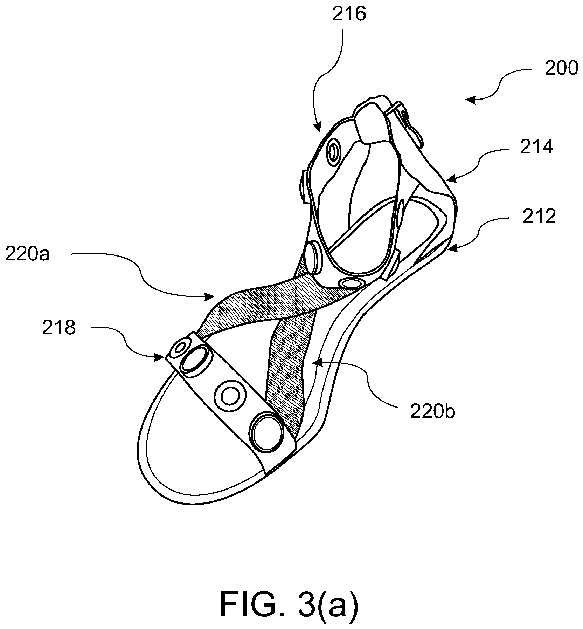

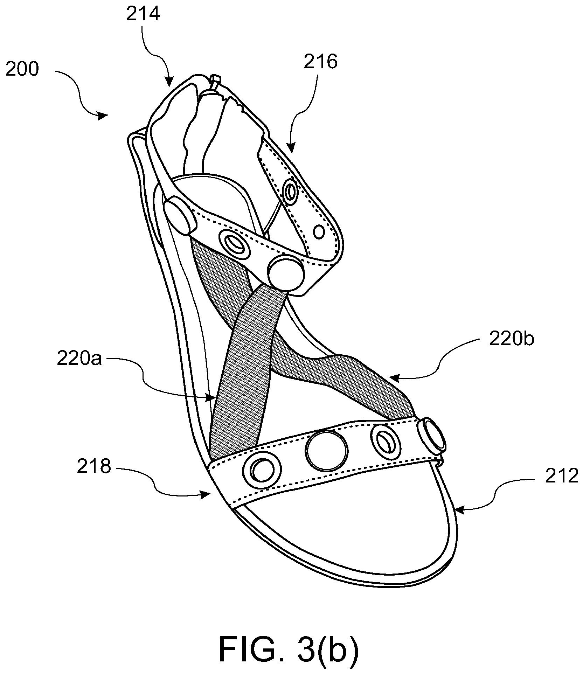

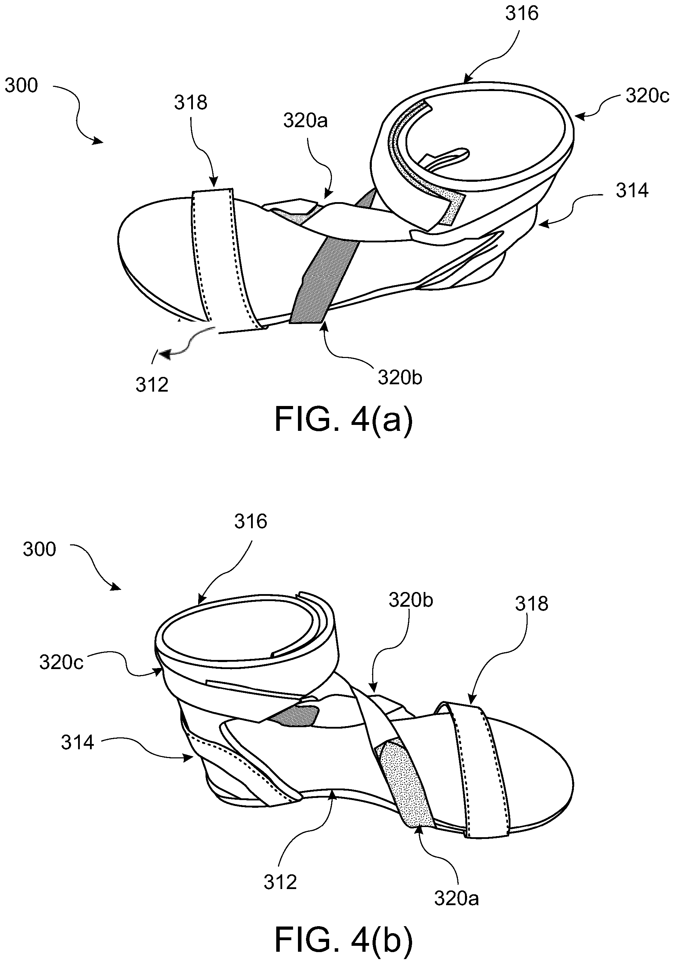

Figure 1 is an illustration of an article of footwear, in accordance with a first embodiment, that may alleviate and/or reduce symptoms of foot drop. In the present embodiment, the article of footwear is a shoe 10. The shoe 10 has a sole member, which is referred to as sole 12. Figure 1 and Figure 2 are illustrative examples of an article of footwear, in accordance with embodiments. Figures 1 and 2 illustrate features of the article of footwear. Although referred to as a shoe, Figure 1 and Figure 2 may be considered to depict either a standalone shoe or part of a skeleton or base for manufacturing a shoe. Figures 3(a) and 3(b) show an embodiment, in which the article of footwear is a sandal. Figures 4(a) and 4(b) depict further embodiments, in which a skeleton or base of a shoe is described.

The sole 12 can generally be described as having a forward portion, a middle portion and a backward portion. In use, it will be understood that, for a correctly sized shoe, the backward portion corresponds to the portion of the sole that seats the heel of a received foot and the forward portion corresponds to the portion of the sole that seats the toes of the received foot. Therefore, the forward and backward portions of the sole may be referred to as the toe and heel portions of the sole.

In use, the middle portion corresponds to the portion of the sole 12 that seats the ball of the foot and the arch of the foot. The middle portion has a first, forward middle portion and a second, rear middle portion. In use, the forward middle portion corresponds to the portion of the sole 12 that seats the ball of a received foot. The forward middle portion may therefore be referred to as the ball portion of the sole 12. In use, the rear middle portion corresponds to the portion of the sole 12 that seats the arch of a received foot. The rear middle portion may therefore be referred to as the arch portion of the sole 12.

In the present embodiment, the ball portion of the sole 12, provided as part of the middle portion of the sole, is the widest part of the shoe. In a correctly sized shoe, the ball of a received foot will sit at the ball portion of the sole 12.

The shoe 10 has a heel member 14 that is connected to the heel portion of the sole 12. At the upper level of the heel member 14 there is provided an ankle collar 16. In the present embodiment, the ankle collar 16 and heel member on 14 are integrated as a single rear portion. The ankle collar 16 acts as an anchor between the wearer and the shoe. In the present embodiment, the single rear portion provides support to the ankle of the wearer. In the present embodiment, a toe strap 18 is also provided attached at the toe portion of the sole 12, however, it will be understood that a toe cap or other suitable toe retaining means may be provided, in other embodiments,

The shoe 10 has a pair of resilient members 20 including a first resilient member 20a and a second resilient member 20b. In the present embodiment, each of the pair of resilient members 20 are fixedly coupled between the ankle collar 16 and the ball portion of the sole 12. Each of the pair of resilient members 20 are biased against movement of the sole 12 away from the ankle of the wearer. In particular, the pair of resilient members 20 are biased against movement of the ball portion of the sole 12 away from a point of reference towards the back of the shoe 10, for example, a point of reference at the ankle collar 16 and/or at the heel member 14 and/or part of the single rear portion.

For the purposes of the following description, the shoe 10 is described as being in a rest configuration. In the rest configuration, in which no forces are applied to the shoe 10, the pair of resilient members are provided, at a first tension. In the present embodiment, the pair of resilient members are made of elastic material. The elastic material can be characterised by one or more parameters, for example, by their elasticity and/or by a value of Young's modulus. In the rest configuration, the ball portion of the sole 12 sits at a first distance from the ankle collar 16 and/or the heel member 14.

For the purposes of the following description, the shoe 10 can be manipulated to be in one or more further configurations, in which the ball portion of the sole 12 is provided at a second, further distance from the ankle collar 16 or the heel member 14. However, it will be understood that the following manipulations may not occur during actual use or wear but are described to illustrate the functioning of the shoe.

As a first example, movement of the ball portion of the sole 12 from the ankle collar 16 and/or the heel member 14 can result from a rotation of the sole 12 relative to the heel member 14 thereby to place the sole 12 in a rotated configuration. In the rotated configuration, the ball portion is at a second distance from the ankle collar 16. The rotation can be characterised by an increased angle between the sole 12 and the heel member 14. When held in the rotated configuration, the pair of resilient members 20 are at a second tension that is increased relative to the first tension in the rest configuration.

As a second example, movement of the ball portion of the sole 12 may also result from a flexing of the sole 12. This movement moves the sole 12 from the rest configuration to a flexed configuration, respectively. In the flexed configuration, the ball portion of the sole 12 is provided at a third distance from the heel member 14 or from the ankle collar 16 that is less than the first distance in the rest configuration. When the sole 12 is held in the flexed configuration, the pair of resilient members 20 are at a third tension that is increased relative to the first tension of the rest configuration.

As a third example, movement of the ball portion of the sole 12 may also result from a bending of the sole 12. This movement moves the sole 12 from the rest configuration to a bent configuration, respectively. In the bent configuration, the ball portion of the sole 12 is provided at a fourth distance from the heel member 14 or from the ankle collar 16 that is greater than the first distance in the rest configuration. When the sole 12 is held in the bent configuration, the pair of resilient members 20 are at a fourth tension that is increased relative to the first tension of the rest configuration.

While the sole 12 is described in the above as being manipulated to be held in further configurations relative to the rest configuration, it will be understood that, in actual use, the first and second resilient members 20a, 20b act to prevent and/or resist such movement of the sole 12 from the heel member 14 and the ankle collar 16. In particular, the first and second resilient members 20a, 20b act to oppose forces from the foot that would otherwise cause movement of the sole 12 in the absence of the resilient members thereby to prevent or substantially reduce movement of the sole 12.

In the present embodiment, the pair of resilient members 20 are provided in a cross-over arrangement. In the present embodiment, a first sole member coupling 22a is provided to couple a first end of the first resilient member 20a to a right hand side of the ball portion of the sole 12. In addition, a first ankle collar coupling 24a is provided to couple a second end of the first resilient member 20a to a left hand forward part of the ankle collar 16. A second sole member coupling 22b is provided to couple a first end of the second resilient member 20b to a left hand side of the ball portion of the sole 12. In addition, a second ankle collar coupling 24b is provided to couple a second end of the second resilient member 20b to a right hand forward part of the ankle collar 16.

The first sole member coupling 22a is provided at a first edge of the sole 12. The second sole member coupling 22b is provided at a second edge of the sole 12. The first and second edges oppose each other. For the shoe 10 depicted in Figure 1 (a shoe for a right foot) the first edge is the edge corresponding to the outer part of the foot and the second edge is the edge corresponding to the inner part of the foot.

As the first ends of the first and second resilient members 20a, 20b are provided on opposing edges of the ball portion of the sole 12 and each of the second ends of the first and second resilient members 20a, 20b are provided on opposing sides of the ankle collar 16, the pair of resilient members the resilient members cross-over. In the present embodiment, the cross-over of the resilient members 20 is at a cross-over position that is rearward of the ball portion and forward of the heel portion of the sole 12. The cross-over position of the resilient members is above the arch portion of the sole 12. In use, the first and second resilient members 20a, 20b of the present embodiment cross over the bridge of a received foot and are in contact with the bridge of the received foot. It will be understood that, in other embodiments, the cross-over position may be above the ball portion or above the heel portion of the sole 12.

The cross-over of the pair of resilient members may be characterised by a projection of the cross-over position onto the sole 12 i.e. the point of the sole 12 above which the resilient member 20a, 20b cross over. In the embodiment of Figure 1 the projection is positioned in the arch portion of the sole 12. In other embodiments, it will be understood that the projection may be in the ball portion or, for example, the heel portion of the sole 12.

The pair of resilient members 20 are biased against movement of the sole 12 that may result from a number of different movements of the foot during a gait cycle. For example, the pair of resilient members may be biased against movements that result from plantar flexion. In particular, any movement of the sole 12 that results from the movement of the front part of the foot and/or the toes downwards such that the foot is further from the shin (for example, a movement typical of a wearer suffering from foot drop). Therefore, in response to plantar flexion of a foot, response forces will travel through the resilient members in an opposing direction. In particular, a first force is exerted in a first direction along the first resilient member 20a from the front right side towards the upper rear left side of the shoe 10 and a second force is exerted along the second resilient member 20b in a second direction from the front left side to the upper rear right side of the shoe 10.

In this embodiment, the first and second resilient members 20a, 20b cross-over and are provided such that the response forces are in substantially perpendicular directions. It will be understood that, in other embodiments, the first and second directions may not be substantially perpendicular and may be at an acute or an obtuse angle. In general, the angle that the resilient members make with each other may vary between different users, as the resilient members will conform to the shape of the foot of the wearer.

In use, the resilient members of the shoe 10 are biased against the movement of the sole 12 from the heel member 14 or the ankle collar 16 to resist or substantially prevent the sole 12 from being moved, for example, into a rotated, bent or flexed configuration during a walking or running cycle.

In comparison to known orthotics, which have a single resilient member, for example, attached at a single coupling point to the top of the foot, such that forces travel through a single point, the cross-over arrangement may act to disperse forces. This may provide comfort benefits. The cross-over arrangement may also prevent further foot movement during gait, for example, movements such as inversion/eversion.

Figures 1 and 2 show an article of footwear or part thereof provided in accordance with an embodiment of the invention. It will be understood that the article of footwear described above may, in some embodiments, be provided as a shoe itself or provided as part of a shoe. For example, the article of footwear may be provided as a base or skeleton on which other parts of the shoes can be built upon to form a finished shoe. One or more components of the article of footwear, for example, the resilient members and/or the ankle collar may be visible during wear.

Figure 2 shows a shoe 100 in accordance with a second embodiment of the invention. Shoe 100 has features corresponding to features of shoe 10. In particular, shoe 100 has a first and second resilient member 120a, 120b corresponding to first and second resilient members 20a, 20b of shoe 10. Shoe 100 also has sole 112, toe strap 118 and heel member 114 which correspond to sole 12, toe strap 18 and heel member 14, respectively, of shoe 10.

Shoe 100 has two resilient members 120a, 120b. The first resilient member 120a is coupled at a first end to a right hand side of the ball portion of the sole 112 and at a second end to the left hand side of a rear part of the ankle collar 116. The second resilient member 120b is coupled at a first end to a left hand side of the ball portion of the sole 112 and at a second end to the right hand side of a rear part of the ankle collar 116. Thus, when worn, the two resilient member 120a, 120b cross over at a bridge of the received foot.

In further detail, the sole member couplings and ankle collar couplings are provided in a different position on shoe 100 in comparison to shoe 10. A first sole member coupling 122a is provided to couple a first end of the first resilient member 120a to a right hand side of the ball portion of the sole 112. In addition, a first ankle collar coupling 124a is provided to couple a second end of the first resilient member 120a to a left hand side part of the ankle collar 116. A second sole member coupling 122b is provided to couple a first end of the second resilient member 120b to a left hand side of the ball portion of the sole 112. In addition, a second ankle collar coupling 124b is provided to couple a second end of the second resilient member 120b to a right hand side part of the ankle collar 116.

The first sole member coupling 122a is provided at a first edge of the sole 112. The second sole member coupling 122b is provided at a second edge of the sole 112. The first and second edges oppose each other. For the shoe 100 depicted in Figure 2 (a shoe for a right foot) the first edge corresponds to the outer part of the foot and the second edge is an edge corresponds to the inner part of the foot.

In contrast to shoe 10, the first ankle collar coupling 124a and the second ankle collar coupling 124b are provided towards the rear of the ankle collar 116 and heel member 114. It will be understood that, in further embodiments, the first and second resilient members 120a and 120b may form part of a single member that is attached at its midpoint to the rear of the ankle collar 116 and the heel member 114. An example of a single member providing both resilient members is provided at Figure 4.

Figures 3(a) and 3(b) show views of an article of footwear, in accordance with embodiments. Figure 3(a) and 3(b) show a sandal 200. Sandal 200 substantially corresponds to the shoe 10 described with reference to Figure 1. In particular, sandal 200 has a sole 212, heel member 214, ankle collar 216, toe strap 218 and first and second resilient members 220a, 220b corresponding to sole 12, heel member 14, ankle collar 16, toe strap 18 and first and second resilient members 20a, 20b as described with reference to Figure 1.

As can be seen in Figures 3(a) and 3(b), ankle collar 216 and toe strap 218 form part of the sandal. These parts are decorated in the present embodiment. The resilient members 220a, 220b, ankle collar 216 and toe strap 218 are visible in use and are provided as part of the sandal design.

Figure 4 is a view of a skeleton 300 or base for a shoe in accordance with a further embodiment. Skeleton 300 for shoe is provided substantially in line with the shoe 10 described with reference to Figure 1, but with modifications, as described in the following. Figure 4 illustrates variations to the resilient members and the ankle collar.

In further detail, skeleton 300 has a sole 312, heel member 314, ankle collar 316 and toe strap 318 corresponding to sole 12, heel member 14, ankle collar 16 and toe strap 18, respectively as described with reference to Figure 1. However, in contrast to the article of footwear of Figure 1, Figures 4(a) and 4(b) depict an embodiment in which the first resilient member and the second resilient member are provided as parts of a single member that is looped round and attached to the rear part of the ankle collar 316. The single member is a single continuous member. The single continuous member wraps, at least in part, around an ankle and has a secured portion that is secured to the ankle collar.

In further detail, the single member has three portions: a first resilient portion 320a, a second resilient portion 320b and a third portion also referred to as an ankle portion 320c. The first resilient portion 320a is provided between the right hand side of a ball portion of the sole 312 and a left hand part of the rear of the ankle collar 316. The second resilient portion 320b is provided between the left hand side of the ball portion of the sole 312 and a right hand part of the rear of the ankle collar 316. The ankle portion 320c is provided between the first resilient portion 320a and second resilient portion 320b and wraps around the back of the ankle collar 316. The ankle portion 320c is attached along its length to the ankle collar 316.

The couplings of the single member to the ankle collar 316 and sole 312 are such that the ankle portion 320c is fixed to the ankle collar 316 and thus the first resilient portion 320a and the second resilient portion 320b are free to move. Thus the first resilient portion 320a and second resilient portion 320b of the single member substantially correspond to the first resilient member 20a and second resilient member 20b as described with reference to Figure 1. In particular, the first and second resilient portions 320a, 320b can be considered to have ankle collar couplings and sole member couplings as described with reference to the shoe 100 of Figure 2.

In some embodiments, the first and second resilient portions 320a and 320b of the single member have a first stretch property and the ankle portion 320c has a second stretch property such that the first and second resilient portions 320a, 320b stretch to a greater degree than the ankle portion 320c. While the single member is shown as a separate component secured to the ankle collar 316, in further embodiments, the single member and the ankle collar may form a single continuous structure.

The ankle collar 316 of skeleton 300 is an adjustable ankle collar. Likewise first and second resilient portions 320a and 320b are also adjustable. In particular, the ankle collar 316, which is wrapped around an ankle of a user, has a hook and loop fastening system to allow the ankle collar to be tightened or loosened. It will be understood that other fastening systems are suitable, for example, a buckle, clasp, zip, button, pin and toggle.

The ankle collar 316 has a diameter and an adjustment mechanism for adjusting the diameter of the ankle collar. As can be seen in Figure 4, the ankle collar has a length that is adjustable thereby to adjust the diameter of the ankle collar. The ankle collar 316 is configured to wrap around the ankle such that, in use, the diameter across the ankle collar is adjustable. In some embodiments, the ankle collar coils around an ankle. In this embodiment, the adjustment mechanism comprises the length of the ankle collar and the fastening system.

Likewise, resilient member portions 320a and 320b have a fitting mechanism allowing the resilient member portions to be tightened or loosened. It will be understood that such an adjustable fitting mechanism can be provided for to embodiments that have separate first and second resilient members. It will be further understood that adjustable resilient members may be provided separately from adjustable ankle collars.

Figure 4 shows a skeleton 300 of a shoe. Figures 1 and 2 may also be considered to depict an underlying skeleton of a shoe or at least features that can form part of an underlying skeleton of a shoe. It will be understood that one or more elements of the depicted skeleton may be integrated into the finished shoe itself. One or more elements of the skeleton may be visible in the finished shoe.

As a first set of non-limiting examples, elements of the depicted shoes may form part of an inner part of the shoe that is configured to be fitted to an outer part of the shoe. As a first example, the sole member, for example the sole 12 of the shoe 10 depicted in Figure 1 may form part an inner sole of a shoe. The resilient members may be provided inside and/or may be integrated as part of the outer shell of the shoe. Alternatively, the sole may form part of an outer sole of the article of footwear. As a further example, the heel member forms part of an inner shoe, for example, the heel counter of a shoe. As a further example, the ankle collar may form part of an inner shoe.

As a further set of examples, the article of footwear may be manufactured from at least an upper and a sole. In such examples, one or more of the described elements (for example, ankle collar, resilient members, toe strap, heel member) are integrated into an upper of the article of footwear. During a manufacturing process, the upper may be manufactured into the one or more resilient members, as a separate part of the shoe before being attached to the sole to form the finished shoe. The one or more resilient members and/or the ankle collar may be integrated into the upper of the shoe. The heel member may also form part of the upper.

In a further example, the toe strap forms part of or is replaced by or is provided inside a toe cap, for example, a toe cap of a boot. As a further example, the one or more resilient members comprise or form part of a saddle of the shoe. As a further example, the ankle collar could provide part of a cuff or topline of the article of footwear.

When manufacturing the article of footwear, an upper may be manufactured, in accordance with a further embodiment. The upper is then attached to a sole as part of the manufacturing process. The upper has an ankle collar, a heel member and one or more resilient members coupled to the ankle collar, substantially as described with reference to the above embodiments. As part of the manufacturing process, the upper is attached to the sole member. As part of the attachment process, the one or more resilient members are attached to the ball portion of the sole.

In the above-described embodiments, a shoe or other article of footwear for use by persons experiencing foot drop was described. In addition to use as an orthotic, in further embodiments, there is provided an athletic shoe for use in sports or other athletic activities or leisure activities, for example, outdoor walking.

During running or walking, a person is prone to symptoms that may be similar to foot drop. In particular, the muscle at the front of the shin (tibialis anterior) which is one of the muscles used to keep the foot up may become fatigued.

An article of footwear for athletic or leisure purposes, in accordance with further embodiments, may be substantially the same as the articles of footwear described in the above embodiments. In certain embodiments, there may be differences between an article of footwear provided for orthotic purposes and an article of footwear provided for athletic or leisure purposes. These differences concern modifications of the shoe for use in athletic or leisure activities.

As a first example, in the above-described embodiments, the resilient members are described as made of elastic material that have sufficient elasticity to provide resilience in response to forces typical of those experienced with foot drop. With regard to the athletic footwear application, the forces required to compensate for fatigue is less than those required for foot-drop. Therefore, for athletic or leisure applications, for example, for a running shoe, the elastic material may be of a lower grade than the elastic used for orthotic purposes. The resilient members may be made of a less durable or a thinner elastic material, which may be easier to integrate into a shoe or allow a lighter weight of shoe which is more suitable for sports or other leisure purposes.

As a second example of a suitable modification, the one or more resilient members may be replaced by a mesh or other material that provides resilience but may also provide breathability and/or be more lightweight. The mesh may be shaped such that it covers the upper part of a received foot and/or forms part of the shoe upper and/or part of an inner or intermediate part of the shoe. In some embodiments, the mesh conforms to the upper part including the arch of a received foot.

As a third example, the athletic shoe may include further adjustable elements. The adjustable elements may include the resilient members and/or the ankle collar. The adjustable elements may allow for adjustment in response to over and/or under pronation during running or walking. In contrast to known trainers, the ankle collar may be integrated into the upper of the athletic shoe, for example, to provide part of the cuff or top-line of the shoe.

In the above described embodiments, the one or more resilient members may be biased against movement of the sole member from the ankle collar. It will be understood that in some embodiments, the movement may comprise lateral movement of at least part of the sole member, for example, in a transverse plane. In these embodiments, the one or more resilient members may be biased against lateral movement of at least part of the sole member. For example, the one or more resilient members may be biased against movement of the sole member in a transverse plane. Thus, in use, the one or more resilient members may prevent or reduce adduction and abduction in a transverse plane.

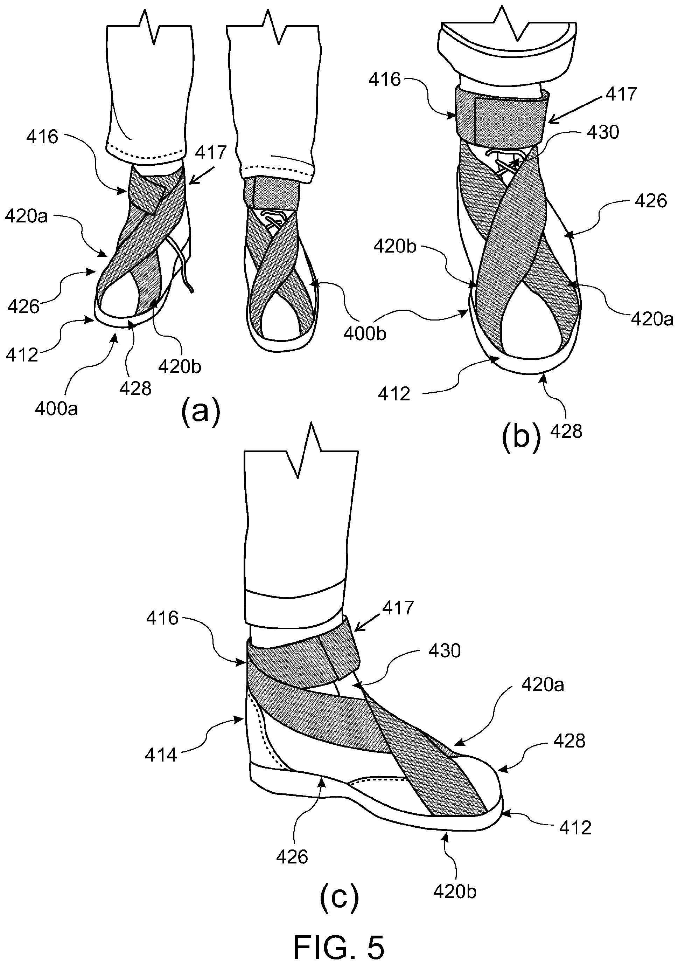

Figure 5(a) to (c) depicts views of a pair of shoes 400a, 400b in accordance with a further embodiment. Figure 5(a) depicts a right shoe 400a for a right foot and a left shoe 400b for a left foot. The shoes 400a, 400b are provided substantially in line with the shoe 10 described with reference to Figure 1. It will be understood that shoe 400a and 400b have the same features, as described in the following.

In further detail, shoe 400a has a sole 412 corresponding to sole 12. In contrast to the previously described Figures, shoe 400a has an upper member 426 (also referred to simply as an upper). The upper 426 is attached to the sole 412 using known methods. The upper 426 incorporates a heel member 414, which corresponds to the heel member 14 described with reference to Figure 1. In place of a toe strap the upper provides has a toe portion, also referred to as a toebox 428. Part of the upper member 426 that spans over the foot is made of a mesh material. Other materials may be used, for example, one or more of: leather, suede, synthetics materials, rubber, foam, EVA, leatherboard, may be used. In this embodiment, the shoe has first and second resilient members 420a and 420b that correspond to first and second resilient member 320a, 320b described with reference to Figure 4.

Figures 5(a) to 5(c) depict a fastening apparatus 430 for fastening each of the shoes 400a, 400b to a foot. In this embodiment, the fastening apparatus 430 comprises a set of eyelets provided in the upper 426 configured to receive laces. In this embodiment, the eyelets are provided under the first and second resilient members 420a and 420b. It will be understood that other known fastening mechanisms may be used, for example, a hook and loop fastening, or a lace-toggle system.

Figure 5(b) and (c) depict left shoe 400b which has corresponding features. In particular, Figure 5(c) depicts a side view of shoe 400b showing the heel member 414 integrated as part of the upper 426. The heel member comprises a reinforced material compared to, for example, the material of the upper. The heel member may be made, for example, from one or more of leather, suede, synthetics materials, rubber, foam, EVA, leatherboard. The choice of material will depend on, for example, the shoe design and shoe cost.

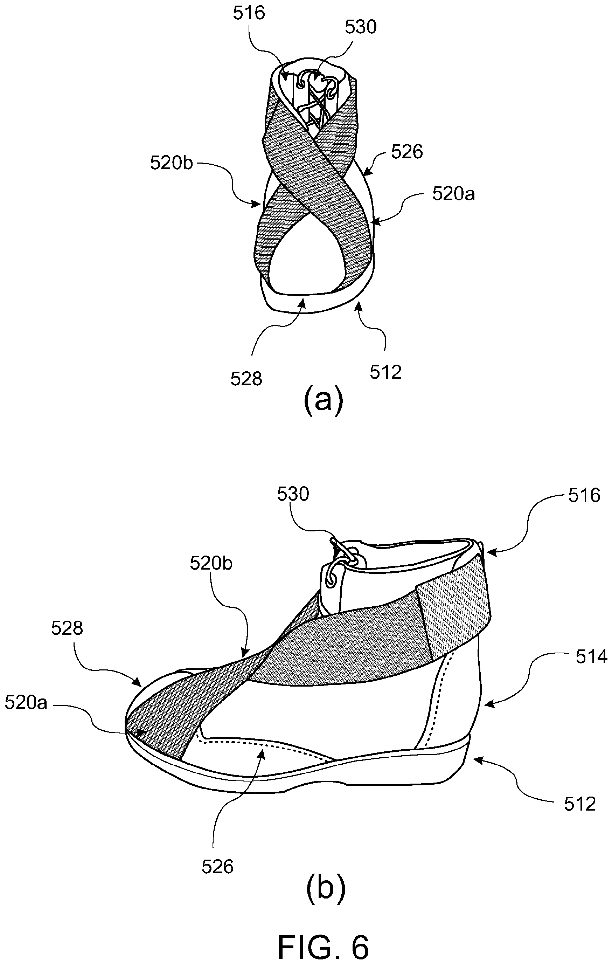

Figures 5(a) to 5(c) also depict an ankle collar 416. The ankle collar is an integrated ankle collar 416 that forms part of the upper 426. The ankle collar 416 provides part of the cuff or top-line of the shoe 400a (or 400b). The ankle collar is reinforced by a further strap 417, which may also be referred to as a reinforcement strap or further ankle strap. As the further strap sits at the same position (and overlapping at least in part) as the integrated ankle collar 416, the integrated ankle collar 416 is not visible and therefore hidden from view in Figure 5. However, Figure 6, described later, depicts substantially the same shoe without the further strap such that the integrated ankle collar (in that Figure referred to as integrated ankle collar 516) is visible.

In this embodiment, a fastening apparatus 430 is provided that is operable to both fasten the shoe to a foot and to adjust the integrated ankle collar 416 to be attached to an ankle, thereby to fix the integrated ankle collar 416 to the ankle. In this embodiment, the integrated ankle collar 416 substantially surrounds the ankle and is adjustable, using the single fastening apparatus 430, to be fixed to the ankle, and act as an anchor.

The fastening apparatus 430 in this embodiment comprises a plurality of eyelets for receiving a lace. Laces provided in the eyelets may be tied or otherwise retained in place, for example, using a toggle. In this embodiment, a pair of the eyelets is provided in the upper is provided at the ankle collar i.e. at the cuff of the upper. The integrated ankle collar 416 has a gap at the front where the fastening apparatus is provided, however, the fastening apparatus, when tightened, reduces the size of this gap, thus securing and fixing the ankle collar to the ankle. In use, a user tightens the fastening apparatus to tighten the integrated ankle collar 416 around the ankle and to fasten the shoe 500 to the foot. It will be understood that other fastening apparatuses may be used, in other embodiments.

In this embodiment, the further strap 417 is also adjustable and can be fastened in place using a further fastening mechanism. The further fastening mechanism may be any suitable fastening mechanism, for example, a hook and loop fastening mechanism or any suitable fastening mechanism described with reference to the ankle collar of Figure 4(a) and 4(b).

In this embodiment, the fastening apparatus 430 forms part of the fastening mechanism of the integrated ankle collar 416 such that the size of the ankle collar 416 (i.e. the tightness/looseness) is adjusted when fastening the shoe to the foot. In other embodiments, the fastening apparatus 430 is provided separately from the fastening mechanism of the ankle collar 416 such that the size of the ankle collar (i.e. the tightness/looseness) can be adjusted independently of fastening the shoe to the foot. In this embodiment, the fastening apparatus 430 is provided independently of fastening mechanism of the further strap 417.

In use, a wearer places their foot in the shoe and adjusts the fastening apparatus 430 to fasten the shoe to their foot and to tighten the integrated ankle collar 416 to their ankle. In particular, the size of the integrated ankle collar 516 is fixed to match the size of their ankle. The user then wraps further strap 417 around their ankle of the wearer and fastens further strap 417 using the fastening mechanism of the further strap. In the present embodiment, the further strap 417 acts to reinforce the integrated ankle collar 416, to provide further strength and to protect integrated ankle collar 416. While both the further strap 417 and the integrated ankle collar 416 may be considered to fix the shoe to the ankle, together they can be considered as fixing the shoe to the ankle and provide an anchor point between the shoe and the ankle.

In addition, as described with reference to Figure 4(a) and 4(b), the first resilient member 520a and the second resilient member 520b are provided as parts of a single member that is looped round and attached to the rear part of the ankle collar 516. The single member is a single continuous member. The single continuous member wraps, at least in part, around an ankle and has a secured portion that is secured to the ankle collar.

Figure 6(a) and 6(b) depicts a shoe 500 in accordance with a further embodiment in which the ankle collar is integrated into the upper of the shoe. The shoe of Figure 6(a) and 6(b) corresponds substantially to the shoe 400a (or shoe 400b) depicted in Figure 5. In particular, shoe 500 has sole 512, heel member 514, toebox 528, first and second resilient members 520a, 520b substantially as described with reference to Figure 5. In addition, shoe 500 has integrated ankle collar 516 (corresponding to ankle collar 416). In contrast, Figure 6(a) and 6(b) does not depict further strap 417. It will be understood that, in some embodiments, the further strap 417 may not be visible when the shoe is not being worn, for example, the further strap 417 is provided inside the shoe.

The article of footwear may be made of a number of different materials. For example, one or more of leather, nylon, synthetic materials, rubber, foam, EVA, leatherboard, suede and plastic. The material used will dependent on the design/fashion of the shoe and cost. Different materials may be used for different parts of the shoe, and the above materials may be used, for example, for the heel member, the ankle collar, the integrated collar, the further strap and/or the resilient members.

By integrating the above-described features into an article of footwear, an article of footwear that performs to alleviate and/or reduce symptoms of foot drop while being inconspicuous may be provided. In further embodiment, certain features of the article of footwear can be made into design features. By integrating all the different elements into a shoe that can be worn independently of a brace, in addition to providing the required support, the shoe may be more acceptable and comfortable for a user, and be easier to put on.

Furthermore, in contrast to known braces and splints, the shoe is provided as a single wearable item. The shoe itself may biomechanically correct/minimise pathological gait associated with drop foot without using a further assistive item (in contrast to known options of known splints/braces that are designed to be worn with a regular shoe).

In the above embodiments, an ankle collar with an adjustment mechanism is described. It will be understood that the ankle collar may be fixedly attachable to the ankle and therefore act as an anchor between the ankle and the shoe, by adjusting and fixing the size of the ankle collar. The size of the ankle collar may be adjustable, for example, to adjust the radius and/or length. The ankle collar may have an adjustment and/or fixing mechanism allowing a user to adjust the ankle collar to a desired size and then fix the ankle collar at the desired size. The desired size may be one in a continuum of sizes or one of a pre-set size. The choices of sizes may correspond to typical sizes of an ankle.

A skilled person will appreciate that variations of the enclosed arrangement are possible without departing from the invention. For example, in the above-described embodiments, articles of footwear such as shoes, trainers and sandals are described. However, it will be understood that the above-described features of the above embodiments, may be provided as part of other types of footwear. As non-limiting example, the article of footwear may be a boot, a slipper, a running shoe, a hiking boot and/or a specialized sports shoe.

As a further non-limiting example, the pair of resilient members have adjustable tension mechanism allowing the tension of the resilient members to be adjusted. Such a mechanism may be a buckle or toggle, for example or other suitable mechanism allowing the length of the resilient members to be adjusted and secured at the adjusted length. The un-stretched length is therefore fixed. Such a mechanism may include fixing the resilient member at one or more points along its length. Such a mechanism may comprise, for example, buckle, clasp, zip, button, pin and toggle. In some embodiments, each resilient member of the pair of resilient members has a corresponding adjustable tension mechanism allowing the tension of each member to be adjusted independently. This feature may allow different tensions to be distributed over the foot to compensate for lack of muscle strength.

As a further non-limiting example, it will be understood that the functionality of the shoe may be optional to some degree. For example, if a person has unilateral foot drop (i.e. foot drop in a single foot only) they do not adjust the adjustable straps to tighten the ankle collar as tightly on their non-footdrop side. In alternative embodiments, it will be understood that the article of footwear may be provided in a pair of shoes.

It will be understood that is some embodiments, the material used may be dependent on the design/fashion of the shoe, for example, leather, suede, synthetics materials, rubber, foam, EVA, leatherboard.

Accordingly, the above description of the specific embodiment is made by way of example only and not for the purposes of limitations. It will be clear to the skilled person that minor modifications may be made without significant changes to the operation described.

## Claims

1. A shoe comprising:
a sole member comprising a toe portion, a ball portion and a heel portion;
an ankle collar configured to be fixed to an ankle;
a heel member configured to at least partially surround a heel of a foot, the heel member extending between the heel portion of the sole member and the ankle collar;
one or more resilient members fixedly coupled between the ankle collar and substantially the ball portion of the sole member, wherein the one or more resilient members are biased against movement of the sole member away from the ankle collar and wherein the one or more resilient members are provided in a cross-over arrangement.

2. A shoe as claimed in claim 1, wherein the movement of the sole member comprises movement of the sole member from a rest configuration towards a further configuration such that in the rest configuration the one or more resilient members are at a first tension and such that, when held in the second position, the one or more resilient members are at a second, increased tension.

3. A shoe as claimed in claims 1 or 2, wherein the cross-over arrangement is such that the one or more resilient members cross-over at a cross-over position above an area of the sole member between the ball portion and the heel portion of the sole member.

4. A shoe as claimed in any preceding claim, wherein each of the one or more resilient members comprises an adjustable tension mechanism allowing the tension of each resilient member to be adjusted independently.

5. A shoe as claimed in any preceding claim, wherein the one or more resilient members are provided such that, in response to a movement of the sole member away from the ankle collar, the one or more resilient members provide one or more forces opposing said movement, optionally wherein the one or more resilient members are arranged to disperse the one or more forces produced in response to movement of the sole member away from the ankle collar, optionally, wherein the one or more produced forces are substantially along the one or more resilient members.

6. A shoe as claimed in any preceding claim, wherein the one or more resilient members comprise at least two resilient members wherein a first member exerts a first force in response to movement of the sole member away from the ankle collar and the second member exerts a second force in response to movement of the sole member away from the ankle collar, optionally, wherein the first force is produced in a first direction and the second force is produced in a second direction, wherein the first direction is substantially perpendicular to the second direction.

7. A shoe as claimed in any preceding claim, where the one or more resilient members comprise an elastic material, optionally wherein the resilient members comprise medical grade elastic and/or wherein the one or more resilient members are further biased against lateral movement of at least part of the sole member and/or wherein the one or more resilient members comprise of or comprise part of a mesh.

8. A shoe as claimed in any preceding claim, wherein at least one of the ankle collar and/or at least one of the one or more resilient members is adjustable and/or wherein the ankle collar comprises a diameter and an adjustment mechanism for adjusting the diameter of the ankle collar.

9. A shoe as claimed in any preceding claim, wherein the heel member has a length extending substantially perpendicular to the sole member such that the ankle collar is provided at a fixed distance from the heel portion of the sole member wherein the length is such that, in use, the ankle collar is provided substantially at an ankle of a wearer.

10. A shoe as claimed in any preceding claim, further comprising at least one of a) and b):
a) one or more sole member couplings between the one or more resilient members and the sole member, wherein the one or more sole member couplings is provided at the ball region of the sole member;
b) one or more ankle collar couplings between the one or more resilient members and the ankle collar, wherein the one or more ankle collar couplings are provided at one or more of a front, back and/or a side of the ankle collar.

11. A shoe as claimed in any preceding claim, wherein at least one of:
a) the sole member comprises or forms part of an inner sole of the shoe;
b) the sole member comprises or forms part of an outer sole of the shoe;
c) the one or more resilient members comprise or are integrated into an upper of the shoe;
d) the one or more resilient members comprise or form part of a saddle of the shoe;
e) the ankle collar is integrated into the upper of the shoe;
f) the toe strap comprises or forms part of a toe cap;
g) the ankle collar comprises or provides part of a cuff or topline of the shoe;
h) the heel portion comprises or forms part of the upper and/or a heel counter of the shoe;
i) the heel portion comprises or forms part of an inner or an outer part of the shoe;
j) the shoe is at least one of a boot, a sandal, an athletic shoe, a running shoe and/or a hiking boot..

12. A shoe as claimed in any preceding claim, wherein the ankle collar comprises or provides part of a cuff or topline of the shoe and the shoe further comprises a further strap configured to be wrapped around an ankle, wherein the further strap acts to reinforce the ankle collar.

13. An upper for a shoe, wherein the upper is configured to be attached to a sole member comprising a toe portion, a ball portion and a heel portion, wherein the upper comprises:
an ankle collar configured to be fixed to an ankle;
a heel member configured to partially surround a heel of a foot, wherein the heel member extends from the ankle collar and is configured to be attached to a heel portion of a sole member such that, when assembled, the heel member is extending between the heel portion of the sole member and the ankle collar; wherein the upper further comprises:
one or more resilient members coupled to the ankle collar and configured to be coupled to substantially the ball portion of the sole member, such that, when assembled, the one or more resilient members are biased against movement of the sole member away from the ankle collar and are provided in a cross-over arrangement.

14. A kit of parts comprising, the upper as claimed in claim 19 and a sole member comprising a toe portion, a ball portion and a heel portion.

15. A method of assembling a shoe comprising:
providing an upper as claimed in claim 13;
providing a sole member comprising a toe portion, a ball portion and a heel portion;
attaching the upper to the sole member, wherein attaching the upper to the sole member comprises:
attaching the heel member of the upper to the heel portion of the sole member;
coupling the one or more resilient members to a ball portion of the sole member such that the one or more resilient members are provided in a cross-over arrangement.
